# EUROPEAN PATENT APPLICATION

(11) **EP 4 129 478 A1**
(43) Date of publication of application: **08.02.2023**
(21) Application number: 21198477.8
(22) Date of filing: 23.09.2021
(51) Int. Cl.: B01L 3/00, C12M 1/26, C12M 1/00, A61M 1/36

(54) **CHAMBER FOR CENTRIFUGAL SEPARATION AND METHOD OF CENTRIFUGAL SEPARATION USING THE SAME**

(30) Priority: 03.08.2021 KR 20210030108
(71) Applicant: Cytodx Inc, Daejeon 34141 (KR)
(72) Inventor: SUNOO, Joseph, Gyeonggi-do (KR); HONG, Min Pyo, Gyeonggi-do (KR); RYU, Sin Uk, Seoul (KR)
(74) Representative: Isarpatent

(57) **Abstract**

A chamber for centrifugal separation includes a chamber body having an upper opening and a lower opening opposite to the upper opening, an upper cover having an upper injection port and mounted on a chamber main body to cover the upper opening, a lower cover having a lower injection port and mounted on the chamber body to cover the lower opening, a receiving space provided in the chamber main body, and including a first receiving part communicating with the lower injection port and an extracting part extending upward from the first receiving part and having a section area smaller than a sectional area of the first receiving part, and a remaining liquid receiving space provided in the chamber main body to receive a material flowing over the receiving space, as the material is injected into the receiving space from the lower injection port.

## Description

### BACKGROUND

### 1. Field

The disclosure relates to a chamber for centrifugal separation and a method for centrifugal separation using the same.

### 2. Description of Related Art

A centrifuge may be used to extract peripheral blood mononuclear cells (PBMCs) or circulating tumor cell (CTCs) from blood. However, a remarkably small number of PBMCs or the CTCs are present in the blood, and, if the PBMCs or the CTCs are not separated within 24 hours after the blood of a person is collected, the cells may be destroyed. Accordingly, the PBMCs or the CTCs should be rapidly and exactly extracted.

Conventionally, after injecting a suspended density gradient material and blood into a container, such as a conical tube, and centrifuging the result, an extraction tool, such as a pipette, is inserted to a position, at which the separated PBMCs are placed, to extract the PBMCs. However, as the suspended density gradient material and the blood are mixed before the centrifugal separation, PBMCs or CTCs may be easily lost. In addition, because a person has a limitation in exactly inserting the extraction tool to the position, at which the PBMCs are placed, through a manual work, it is difficult to quantatively extract the PBMCs or the CTCs.

In addition, an amount of PMC or CTC contained in the blood of the person is varied depending on persons. Accordingly, a necessary amount of blood for extracting a required amount of PBMC or CTC is varied depending on the persons. Accordingly, it may be inappropriate to extracting the PBMC or CTC from blood of various types of people by using a fixed-size vessel.

### SUMMARY

Aspects of the disclosure are to address at least the above-mentioned problems and/or disadvantages and to provide at least the advantages described below. Accordingly, an aspect of the disclosure is to provide a chamber for centrifugal separation, capable of easily extracting a target material, which is to be extracted, and a method for centrifugal separation using the same.

In accordance with another aspect of the disclosure, a chamber for centrifugal separation includes a chamber body having an upper opening and a lower opening opposite to the upper opening, an upper cover having an upper injection port and mounted on a chamber main body to cover the upper opening, a lower cover having a lower injection port and mounted on the chamber body to cover the lower opening, a receiving space provided in the chamber main body, and including a first receiving part communicating with the lower injection port and an extracting part extending upward from the first receiving part and having a sectional area smaller than a sectional area of the first receiving part, and a remaining liquid receiving space provided in the chamber main body to receive the material flowing over the receiving space, as a material is injected into the receiving space from the lower injection port. The remaining liquid receiving space has an observing region to expose at least a portion of a region of the extracting part in a direction (extending direction) that the extracting part extends, when the extracting part is viewed from an outside of the chamber main body.

According to another embodiment, the remaining liquid receiving space may extend in the extending direction of the extracting part, and may be formed to surround a partial region of the extracting part in a circumferential direction of the extracting part. An observing region may be formed in a remaining region, which is not surrounded by the remaining liquid receiving space, of the extracting part in the circumferential direction of the extracting part, in the chamber main body.

According to another embodiment, the observing region may include a transparent material.

According to another embodiment, the observing region may include a recess part recessed inward from the chamber main body.

According to another embodiment, an inner surface of the remaining liquid receiving space in a radial direction may be connected with an outer surface of the receiving space in the radial direction. The outer surface of the remaining liquid receiving space in the radial direction may be formed to protrude upward from the receiving space.

According to another embodiment, the receiving space may further include a second receiving part extending upward from the extracting part and having a sectional area greater than a sectional area of the extracting part.

According to another embodiment, the apparatus may further include a filter device interposed between the upper injection port and the lower injection port, in the receiving space to prevent a material, which is injected through the upper injection port, from being mixed with the material injected through the lower injection port.

According to another embodiment, the lower cover may further include a cap including a protruding part protruding inward of the first receiving part when the lower cover is coupled to the chamber main body, and having the lower injection port. The cap may be inserted in the lower injection port in an axial direction to prevent the material injected into the lower injection port from moving toward the upper opening, when a direction parallel to the direction from the upper opening toward the lower opening is defined as the axial direction, and a direction perpendicular to the axial direction is defined as a radial direction.

According to another embodiment, the lower cover may include a lower cover part to cover the lower opening, and a lower side part extending toward the upper opening from a circumference of the lower cover part to cover an outer surface of the chamber main body when the lower cover is mounted on the chamber main body. The lower side part may include a roughness part having a repeated roughness pattern formed along a circumference of an inner surface opposite to the outer surface of the chamber main body. The chamber main body may include a protruding part formed at a position corresponding to a position of the roughness part, and protruding to be engaged with the roughness part, when the lower cover is coupled to the chamber main body.

According to another embodiment, a method for centrifugal separation using a chamber for the centrifugal separation, in which the chamber includes a chamber body having an upper opening and a lower opening opposite to the upper opening, an upper cover having an upper injection port and mounted on a chamber main body to cover the upper opening, a lower cover having a lower injection port and mounted on the chamber body to cover the lower opening, a receiving space provided in the chamber main body, and including a first receiving part communicating with the lower injection port and an extracting part extending upward from the first receiving part and having a section area smaller than a sectional area of the first receiving part, and a remaining liquid receiving space provided in the chamber main body to receive a material flowing over the receiving space, as the material is injected into the receiving space from the lower injection port, the remaining liquid receiving space having an observing region to expose at least a portion of a region of the extracting part in a direction (extending direction) that the extracting part extends, when the extracting part is viewed from an outside of the chamber main body, may include preparing the chamber for centrifugal separation, injecting a density gradient material into the receiving space through the lower injection port, injecting a target material for the centrifugal separation into the receiving space through the upper injection port, performing the centrifugal separation for a target material for the centrifugal separation by rotating the chamber for the centrifugal separation, placing the target material, which is extracted after the centrifugal separation, in the extracting part, by additionally injecting the density gradient material into the first receiving part through the lower injection port, such that a portion of a material received in the receiving space flows over the remaining liquid receiving space, and extracting the target material to be extracted through the upper injection port while determining the position of the target material to be extracted through the observing region.

Other aspects, advantages, and salient features of the disclosure will become apparent to those skilled in the art from the following detailed description, which, taken in conjunction with the annexed drawings, discloses various embodiments of the disclosure.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 is a perspective view illustrating a chamber for centrifugal separation, according to an embodiment of the disclosure;
FIG. 2 is a front sectional view illustrating a chamber for centrifugal separation, according to an embodiment of the disclosure;
FIG. 3 is a exploded perspective view illustrating a chamber for centrifugal separation, according to an embodiment of the disclosure;
FIG. 4 is a perspective view illustrating a chamber main body, according to an embodiment of the disclosure;
FIG. 5 is a perspective view illustrating a chamber main body, according to an embodiment of the disclosure;
FIG. 6 is a perspective view illustrating a lower cover and a cap, according to an embodiment of the disclosure;
FIG. 7 is a partially cut-out perspective view illustrating a lower cover, according to an embodiment of the disclosure;
FIG. 8 is a perspective view a chamber main body coupled to a lower cover, according to an embodiment of the disclosure;
FIG. 9 is a plan view illustrating a lower cover, according to an embodiment of the disclosure;
FIG. 10 is views illustrating a method for centrifugal separation using a chamber for centrifugal separation, according to an embodiment of the disclosure;
FIG. 11 illustrates views to explain a method of centrifugal separation using a chamber for centrifugal separation, according to an embodiment of the disclosure;
FIG. 12 illustrates views to explain a method of centrifugal separation using a chamber for centrifugal separation, according to an embodiment of the disclosure; and
FIG. 13 is a perspective view illustrating a chamber for centrifugal separation, when extracting a target material, which is to be extracted, according to an embodiment of the disclosure.

### DETAILED DESCRIPTION

Hereinafter, various embodiments of the disclosure will described with reference to accompanying drawings. However, those of ordinary skill in the art should understand that the disclosure is not limited to a specific embodiment, and modifications, equivalents, and/or alternatives on the various embodiments described herein can be variously made without departing from the scope and spirit of the disclosure. With regard to description of drawings, similar components may be assigned with similar reference numerals.

In the disclosure, it will be further understood that the terms "have", "can have," "includes" and/or "can include", when used herein, specify the presence of stated features (for example, components such as a numeric value, a function, an operation, or a part), but do not preclude the presence or addition of one or more other features.

In the disclosure, the expressions "A or B", "at least one of A and/or B", "one or more of A and/or B" may include all possible combinations of one or more of the associated listed items. For example, "A or B", "at least one of A and B", or "at least one of A or B" includes all (1) at least one A, (2) at least one B, or (3) at least one "A" and at least one "B".

The wording " -configured to" used in the disclosure can be interchangeably used with, for example, "suitable for", "having the capacity to", "designed to", "adapted to", "made to", or "capable of'. The wording "-configured to" does not refer to essentially "specifically designed to".

The terms in the disclosure are used only for specific embodiments, and the scope of another embodiment is not limited thereto. The terms of a singular form may include plural forms unless otherwise specified. In addition, unless otherwise defined, all terms used in the disclosure, including technical or scientific terms, have the same meanings as those generally understood by those skilled in the art to which the disclosure pertains. Such terms, which are used herein, as those defined in a generally used dictionary are to be interpreted as having meanings equal to the contextual meanings in the relevant field of art, and are not to be interpreted as having ideal or excessively formal meanings unless clearly defined as having such in the disclosure. Even if the terms are defined in the disclosure, the terms should not be interpreted as excluding embodiments of the disclosure if necessary.

The embodiment disclosed herein should be suggested for the convenience of explanation, and should not limit the scope of the disclosure. Accordingly, the technical scope of the disclosure should be interpreted as including all modifications or various changes based on the technical spirit of the disclosure.

Hereinafter, the embodiment of the disclosure will be described in detail. Before the description of the embodiment, terms and words used in the present specification and the claims should not be interpreted as commonly-used dictionary meanings, but should be interpreted as to be relevant to the technical scope of the disclosure based on the fact that the inventor may properly define the concept of the terms to explain the disclosure in best ways.

Therefore, features of the embodiment described in the disclosure are only part of the most exemplary embodiments of the disclosure, and do not represent all technical scopes of the embodiments, so it should be understood that various equivalents and modifications could exist at the time of filing this application.

Throughout the whole specification, when a certain part "includes" a certain component, the certain part does not exclude other components, but may further include other components unless there is a specific opposite description.

Hereinafter, the disclosure will be described in detail.

### Chamber for Centrifuge

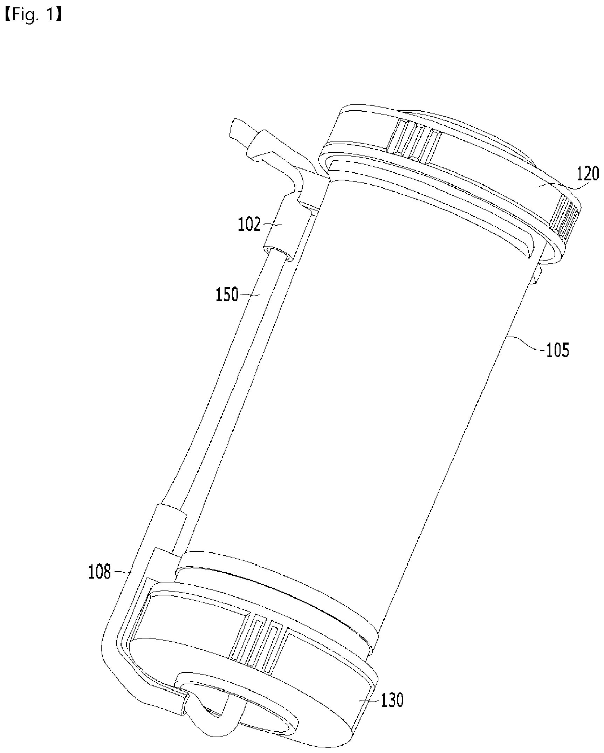

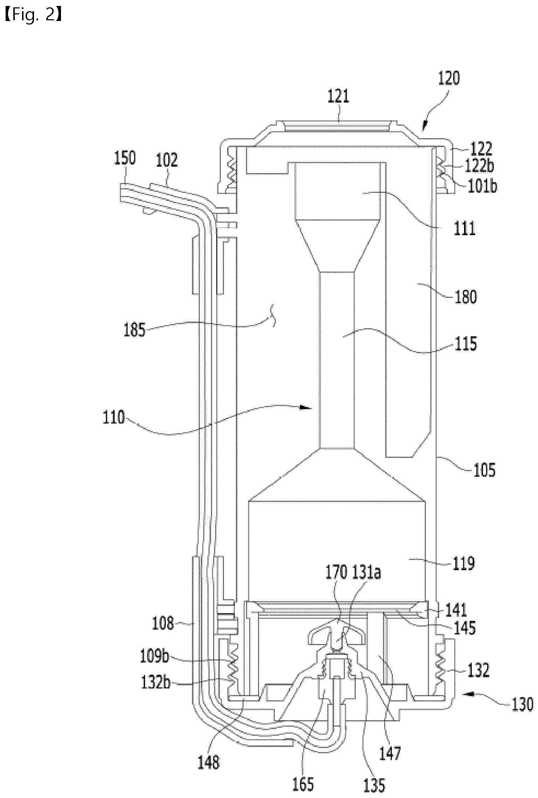

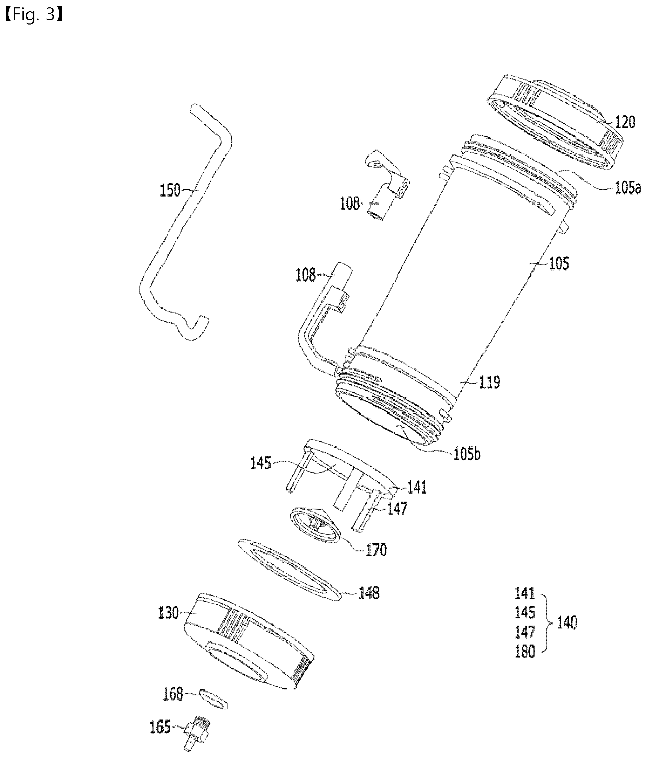

FIG. 1 is a perspective view illustrating a chamber for centrifugal separation, according to an embodiment of the disclosure. FIG. 2 is a front sectional view illustrating a chamber for centrifugal separation, according to an embodiment of the disclosure. FIG. 3 is an exploded perspective view illustrating a chamber for centrifugal separation, according to an embodiment of the disclosure. Hereinafter, a chamber for centrifugal separation will be described with reference to FIGS. 1 to 3, according to an embodiment of the disclosure.

According to an embodiment of the disclosure, the chamber for centrifugal separation includes a chamber main body 105, an upper cover 120, a lower cover 130, a receiving space 110, and a remaining liquid receiving space 180.

The chamber main body 105 may be formed in the shape of a pillar having a hollowed structure. The chamber main body 105 has an upper opening 105a and a lower opening 105b. The upper opening 105a and the lower opening 105b may be provided in opposite ends of the chamber main body 105 while facing each other.

The upper cover 120 has an upper injection port 121a. The upper cover 120 is mounted on the chamber main body 105 to cover the upper opening 105a. Although the upper cover 120 is detachably coupled to the chamber main body 105 through threads, the coupling manner is not specifically limited.

The lower cover 130 has a lower injection port 131a. The lower cover 130 is mounted on the chamber main body 105 to cover the lower opening 105b. Although the lower cover 130 is detachably coupled to the chamber main body 105 through threads, the coupling manner is not specifically limited.

Alternatively, at least one of the upper cover 120 or the lower cover 130 may be provided integrally with the chamber main body 105, and the upper injection port 121a and/or the lower injection port 131a may be provided in the upper portion and/or lower portion of the chamber main body 105.

The receiving space 110 may be provided in the chamber main body 105. The receiving space 110, which is a space to receive a target material for centrifugal separation, communicates with the upper injection port 121a and the lower injection port 131a. In more detail, the receiving space 110 includes a first receiving part 119 communicating with the lower injection port 131a and an extracting part 115 extending upward from the first receiving part 119. Alternative, the receiving space 110 may include a second receiving part 111 extending upward from the extracting part 115.

In this case, the extracting part 115 has a sectional area smaller than the first receiving part 119. The sectional area refers to an area viewed in a radial direction, when a direction parallel to a direction from the upper opening 105a toward the lower opening 105b is defined as an axial direction, and a direction perpendicular to the axial direction is defined as the radial direction. Alternatively, the second receiving part 111 may have a sectional area greater than a sectional area of the extracting part 115. Accordingly, the first receiving part 119 may have the form in which the diameter of the first receiving part 119 is reduced toward the extracting part 115, and the second receiving part 111 may be formed to have a diameter reduced toward the extracting part 115. However, the diameters and the heights, which extend in the axial direction, of the first receiving part 119 and the second receiving part 111 are not specifically limited, but are properly changed depending on the uses of the first receiving part 119 and the second receiving part 111.

Meanwhile, the extracting part 115 may be formed to longitudinally extend in the axial direction. For example, the length of the extracting part 115 in the axial direction may be formed to be equal to or greater than "A" mm and equal to or less than "B" mm, and the diameter of the extracting part 115 may be formed be equal to or greater than "C" mm and equal to or less than "D" mm. The length of the extracting part 115 is formed to be equal to or greater "A" mm to increase an amount of the target material to be extracted, which is received in the extracting part 115. Accordingly, the chamber for centrifugal separation may increase an amount of a material which is receivable. Alternatively, the extracting part 115 longitudinally extends in the axial direction and has a smaller diameter (sectional area). Accordingly, when the target material, which is positioned in the extracting part 115, is extracted, the target material is prevented from being mixed with another material, such that the target material is more exactly extracted.

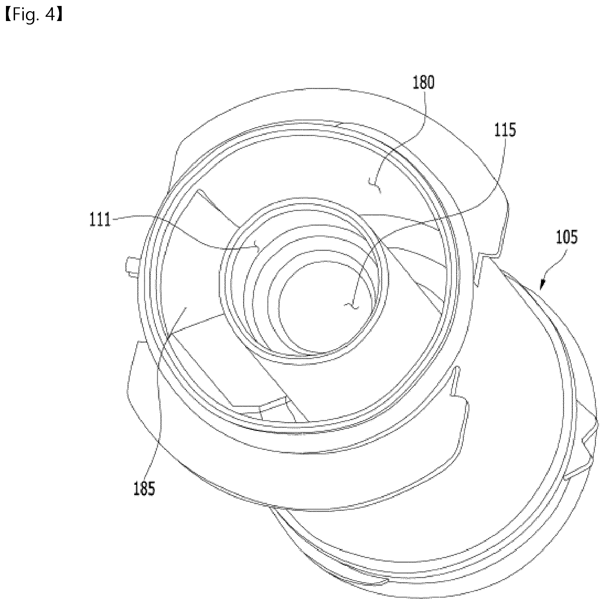

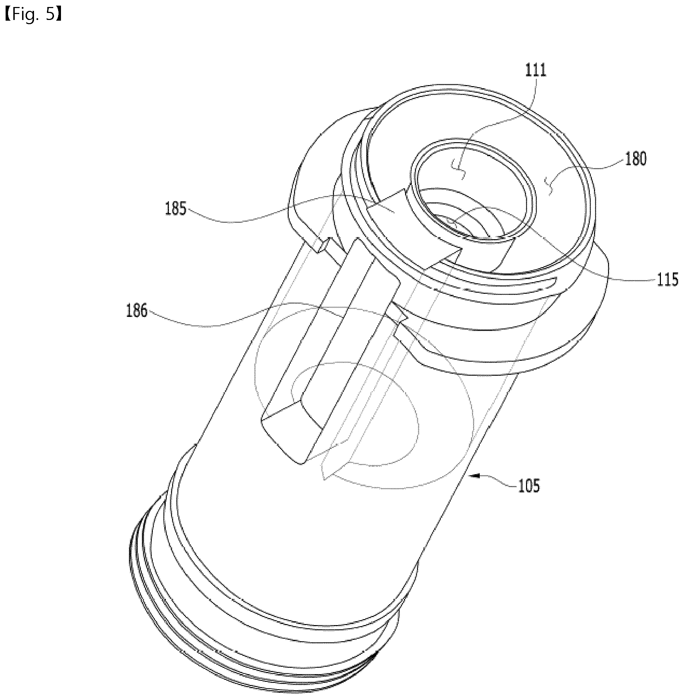

FIG. 4 is a perspective view illustrating a chamber main body, according to an embodiment of the disclosure. FIG. 5 is a perspective view illustrating a chamber main body, according to an embodiment of the disclosure. Hereinafter, the remaining liquid receiving space 180 and an observing region 185 will be described in detail with reference to FIGS. 2, 4, and 5.

The remaining liquid receiving space 180 is provided in the chamber main body 105. The remaining liquid receiving space 180 receives a material flowing over the receiving space 110, as the material is injected into the receiving space 110 from the lower injection port 131a.

In addition, the remaining liquid receiving space 180 has the observing region 185 formed in the chamber main body 105. The observing region 185 refers to a region allowing a user to determine the target material to be extracted, which is positioned in the extracting part 115, when the user views the extracting part 115 from the outside of the chamber main body 105. The remaining liquid receiving space 180 has the observing region 185 to expose at least a portion of a region of the extracting part 115 in a direction (extending direction) that the extracting part 115 extends, such that the user observes the height of the extracting part 115 at which the target material to be extracted is positioned.

For example, the remaining liquid receiving space 180 may have an opening that is open at an upper portion of the remaining liquid receiving space 180, may be formed to extend in the extending direction of the extracting part 115, and may be formed to surround the extracting part 115 in a circumferential direction of the extracting part 115. In addition, the observing region 185 may be formed in a remaining region, which is not surrounded by the remaining liquid receiving space 180, of the extracting part 115 in the circumferential direction of the extracting part 115. In other words, the remaining liquid receiving space 180 surrounds the portion of the extracting part 115 in the circumferential direction. Accordingly, even if a material is received in the remaining liquid receiving space 180, only the portion of the extracting part 115 in the circumferential direction is hidden by the material received in the remaining liquid receiving space 180. Accordingly, the extracting part 115 may be observed through the observing region 185 having not the remaining liquid receiving space 180.

In addition, an inner surface of the remaining liquid receiving space 180 may be connected with an outer surface of the receiving space 110 in the radial direction, at an upper end portion of the receiving space 110. Alternatively, the inner surface of the remaining liquid receiving space 180 in the radial direction may share the same plane with the outer surface of the receiving space 110. Accordingly, a material flowing over the receiving space 110 may be accelerated to rapidly flow into the remaining liquid receiving space 180.

In addition, a top surface of the observing region 185 is positioned to be lower than the upper end portion of the receiving space 110, thereby preventing the material flowing over the receiving space 110 from flowing back into the receiving space 110.

In addition, the outer surface of the remaining liquid receiving space 180 in the radial direction protrudes upward from the receiving space 110, thereby preventing flowing over the receiving space 110 from leaking out of the remaining liquid receiving space 180. Alternatively, the outer surface of the remaining liquid receiving space 180 in the radial direction may be connected with the inner surface of the chamber main body 105, or may share the same plane with the inner surface of the chamber main body 105.

Meanwhile, at least the observing region 185 of the chamber main body 105 may be formed of a transparent material, such that the extracting part 115 is more easily observed. Alternatively, as illustrated in FIG. 5, the observing region 185 includes a recess part 186 recessed inward from the chamber main body 105, such that the extracting part 115 is more easily observed.

Referring back to FIGS. 2 and 3, according to an embodiment of the disclosure, the chamber for centrifugal separation may further include a filter device 140. The filter device 140 serves as an inter-layer boundary provided inside the receiving space 110 to prevent the injected material from being mixed and introduced, and to distribute injection pressure, when the injected material is mixed and introduced into the receiving space 110. Accordingly, the mixture of the material is prevented from being made due to pressure concentrated on a portion of each inter-layer interface when the material is injected. In other words, the present inventors have recognized that the separation of the material having higher purity is performed based on the effect of stabilizing the inter-layer interface, which is produced by distributing the pressure, instead of separating material layers, as the filter device 140 is provided inside the receiving space 110.

The filter device 140 is interposed between the upper injection port 121a and the lower injection port 131a in the receiving space 110. The filter device 140 prevents a material, which is injected into the receiving space 110 through the upper injection port 121a from being mixed with a material injected into the receiving space 110 through the lower injection port 131a. As the filter device 140 is provided to prevent the materials, which are injected through the upper injection port 121a and the lower injection port 131a, from being mixed with each other before centrifugal separation, the target material to be extracted may be smoothly separated after the centrifugal separation.

The filter device 140 includes a filter 145 to filter a material, and a first filter supporting member 141 to mount the filter 145 on the first filter supporting member 141. The position of the first filter supporting member 141 is fixed between the upper injection port 121a and the lower injection port 131a inside the receiving space 110. Accordingly, the filter 145 prevents the material injected through the upper injection port 121a and the material injected through the lower injection port 131a from being mixed with each other.

The filter 145 may be, for example, a mesh filter formed of nylon, polyester, polypropylene, or polyetheretherketone (PEEK). A mesh pore may have a circular shape, an oval shape, or a polygonal shape, and may have the size ranging from 5 *µ*m to 600 *µ*m, but the disclosure is not limited thereto. In addition, the filter 145 is not limited to the mesh filter, but may include various filters, such as a membrane.

Meanwhile, a manner of fixing the position of the first filter supporting member 141 in the chamber axial direction is not specifically limited. According to an embodiment of the disclosure, the filter device 140 includes a plurality of second filter supporting members 147 to support the first filter supporting member 141 such that the position of the first filter supporting member 141 is fixed to a specific position.

In more detail, the second filter supporting member 147 is formed to extend downward from the first filter supporting member 141. The second filter supporting member 147 may have a pillar form, and a plurality of second filter supporting member 147 may be provided. A step part 119a is formed to protrude inward from the inner surface of the chamber main body 105.

Accordingly, when the lower cover 130 is coupled to the chamber main body 105, the second filter supporting member 147 is supported by the lower cover 130 and pressed upward, and the first filter supporting member 141 connected with the second filter supporting member 147 is displaced upward. However, because the step part 119a is formed on the inner surface of the chamber main body 105, a surface, which faces upward, of the first filter supporting member 141 makes contact with the step part 119a, such that the first filter supporting member 141 is blocked from being displaced upward. Accordingly, the first filter supporting member 141 may be fixed in position in the axial direction, between the step part 119a and the second filter supporting member 147. In other words, the second filter supporting member 147 may be formed to have a length supported by the lower cover 130 while the first filter supporting member 141 makes contact with the step part 119a, when the lower cover 130 is coupled to the chamber main body 105.

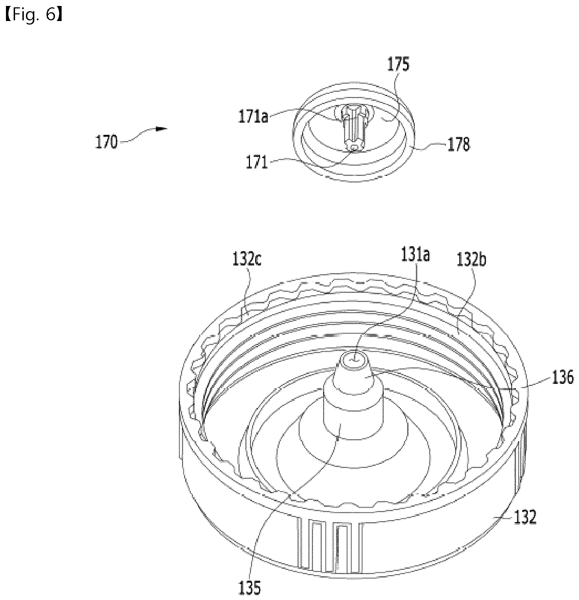

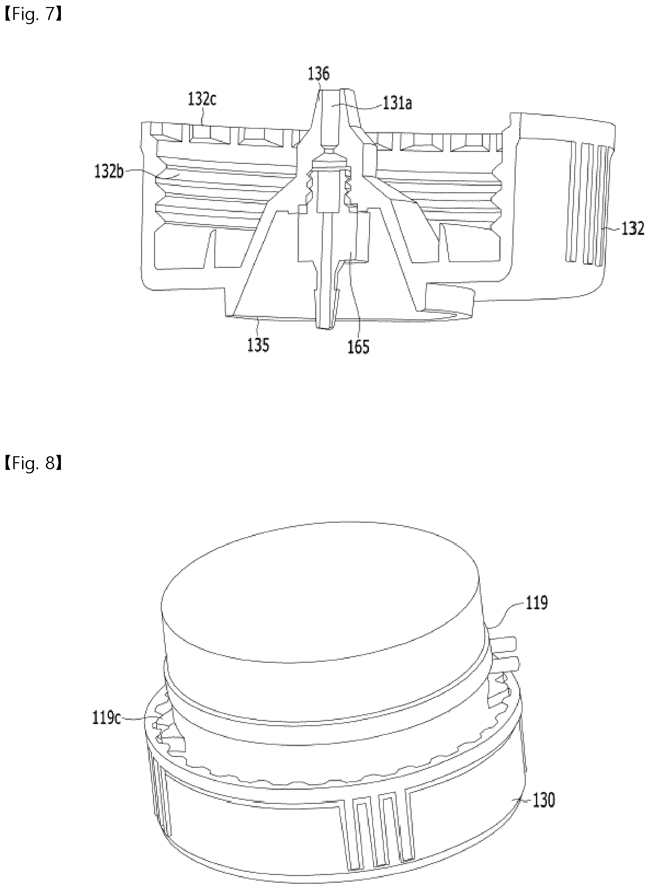

FIG. 6 is a perspective view illustrating a lower cover and a cap, according to an embodiment of the disclosure. FIG. 7 is a partially cut-out perspective view illustrating a lower cover, according to an embodiment of the disclosure. Hereinafter, the upper cover 120 and the lower cover 130 will be described in more detail with reference to FIGS. 2, 3, 6, and 7.

First, referring to FIGS. 2 and 3, the upper cover 120 includes an upper cover part 121 to close an upper opening 105a when coupled to the chamber main body 105, and an upper side part 122 extending from a circumference of the upper cover part 121 to surround an outer surface of the chamber main body 105. The upper cover part 121 includes the upper injection port 121a. In addition, thread parts 122b and 101b to be engaged with each other are formed on an inner surface of the upper side part 122 and on an outer circumferential surface of the chamber main body 105, respectively, such that the upper cover 120 is thread-engaged with the chamber main body 105.

Meanwhile, the target material, such as blood, for centrifugal separation may be injected into the upper injection port 121a or the target material to be extracted after the centrifugal separation may be extracted through the upper injection port 121a. For example, a syringe or pipette may be inserted into the main body 110 through the upper injection port 121a to inject the blood or to extract the separated PBMC.

The lower cover 130 includes a lower cover part 131 to close the lower opening 105b when coupled to the chamber main body 105, and a lower side part 132 extending from a circumference of the lower cover part 131 to surround an outer surface of the second receiving part 111. In addition, thread parts 132b and 109b to be engaged with each other are formed on an inner surface of the lower side part 132 and on an outer circumferential surface of the second main body unit 105, respectively, such that the lower cover 130 is thread-engaged with the chamber main body 105. In addition, a lower cover packing member 148 is interposed between the lower cover part 131 and the second opening 105b to firmly seal the lower opening 105b.

Meanwhile, the lower cover part 131 includes a protruding part 135 protruding inward of the first receiving part 119, when the lower cover 130 is coupled to the chamber main body 105. The protruding part 135 may extend such that a distal end of the protruding part 135 is positioned to be closer to a lower portion, as compared to that the protruding part 135 is close to the filter 145 of the filter device 140. In addition, an extending part 136 is formed in a hollowed structure while extending upward from the distal end of the protruding part 135 (see FIG. 6), and the lower injection port 131a is formed in the extending part 136 in the hollowed structure. Meanwhile, a tube connector 165 may be fitted into the lower injection port 131a while interposing a tube packing member 168 between the tube connector 165 and the lower injection port 131a. The chamber main body 105 may include an upper fixing part 102 and a lower fixing part 108 to fix a tube 150 to the chamber main body 105, and the material injected into the tube 150 is injected into the first receiving part 119 through the tube connection port 165 and the lower injection port 131a.

In this case, a cap 170 is inserted into the lower injection port 131a in the axial direction to prevent the material, which is injected through the lower injection port 131a, from moving upward. Accordingly, even if a material is injected into the lower injection port 131a in the status that a plurality of ingredients are separated in the axial direction after centrifugal separation, the material is prevented from moving upward, such that the plurality of ingredients are prevented from being mixed with each other.

In more detail, referring to FIG. 6, the cap 170 may include a pillar part 171, a base part 175, and a cover part 178. The pillar part 171 refers to a part formed to extend in the axial direction and inserted into the lower injection port 131a. The base part 175 refers to a part formed to extend in the radial direction from an end portion of the pillar part 171. The cover part 178 refers to a part extending downward from a circumference of the base part 175. Accordingly, a fluid passage is formed between an inner circumferential surface of the lower injection port 131a and an outer circumferential surface of the pillar part 171. A material injected through the fluid passage may be injected into the chamber main body along a bottom surface of the base part 175 and an inner surface of the cover part 178 in the radial direction. In other words, the material injected into the lower injection port 131a may be prevented from moving upward.

In this case, a plurality of recess parts 171a are provided in an outer circumferential surface of the pillar part 171 to be recessed inward in the axial direction. Accordingly, a fluid passage may be expanded to inject a larger amount of a material through the lower injection port 131a.

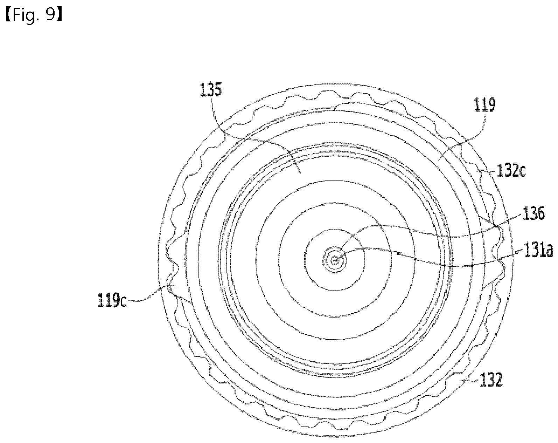

FIG. 8 is a perspective view a chamber main body coupled to a lower cover, according to an embodiment of the disclosure. FIG. 9 is a plan view illustrating a lower cover, according to an embodiment of the disclosure. Hereinafter, a coupling manner and a separating manner between the lower cover 130 and the chamber man body 105 will be described with respect to FIGS. 8 and 9. Although the following description will be made while focusing on the lower cover 130, the substantially same components are applicable to the upper cover 120.

Referring to FIGS. 8 and 9, a roughness part 132c is formed by consecutively arranging a concave part and a convex part in a circumferential direction, on the inner surface of the lower side part 132 of the lower cover 130. In addition, a plurality of protrusion parts 109c are formed to protrude from an outer surface of the chamber main body 105 to be engaged with the roughness part 132c. Although two protruding parts 109c protrude in the shape of "M", the number and the shape of the protruding parts 109c are not specifically limited.

The protruding part 109c is formed at a position corresponding to a position of the roughness part 132c in the axial direction, when the lower cover 130 is coupled to the chamber main body 105. Accordingly, when the lower cover 130 is coupled to the chamber main body 105, the protruding part 109c is engaged with the roughness part 132c having a repeated pattern along the circumference, thereby preventing the lower cover 130 from being separated, that is, rotated.

In addition, the protruding part 109c may be formed of a hard material having relatively less elasticity, and the lower cover 130 may be formed of a flexible material having relatively higher elasticity. Accordingly, when force is applied to the lower cover 130 to couple the lower cover 130 to the chamber main body 105 or to separate the lower cover 130 from the chamber main body 105, the shape of the lower cover 130 is changed (for example, a circular shape is changed to an oval shape) to release the engagement between the protruding part 109c and the roughness part 132c. Accordingly, the coupling and the separation of the lower cover 130 may be easily performed.

### Method for Centrifugal Separation

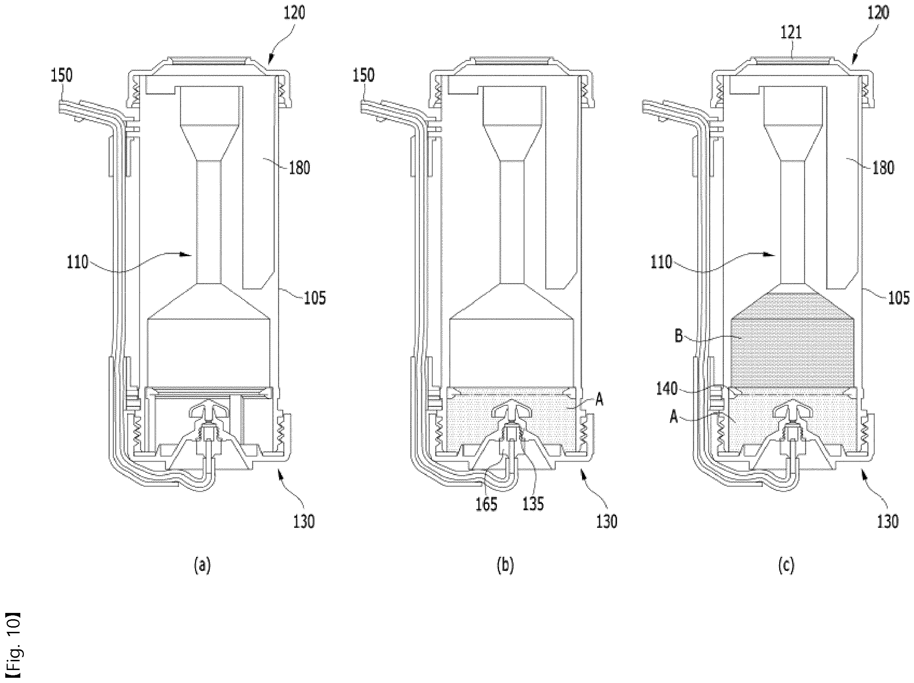

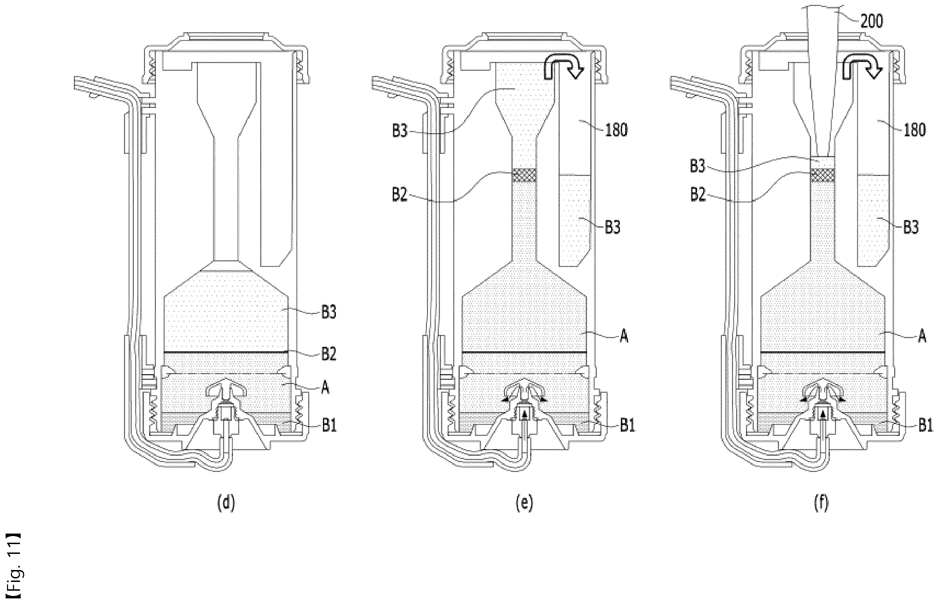

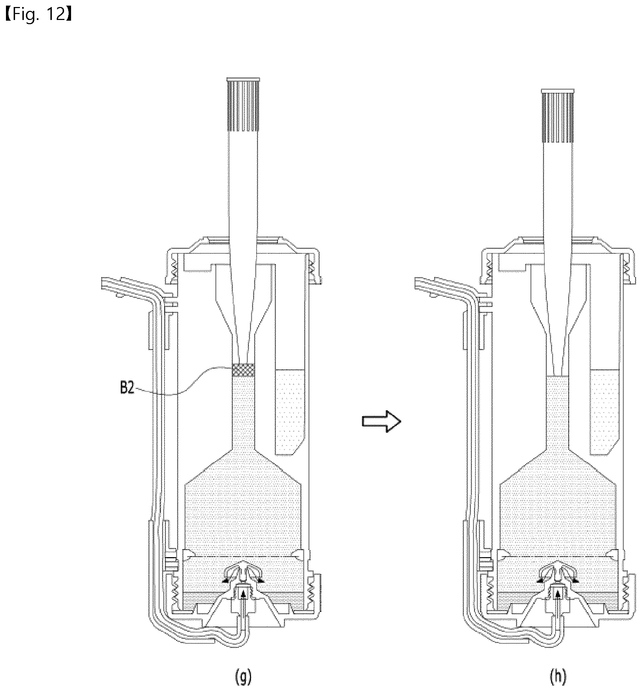

FIGS. 10 to 12 illustrate views to explain a method of centrifugal separation using a chamber for centrifugal separation, according to an embodiment of the disclosure. Reference signs (a) to (c) of FIG. 10 are views illustrating a procedure before centrifugal separation, and reference signs (d) to (f) of FIG. 11 and reference signs (g) and (h) of FIG. 12 are views illustrating a procedure after the centrifugal separation. The following description will be made regarding a manner of extracting a PBMC from blood by performing the centrifugal separation for the blood. However, a target material for centrifugal separation and a target material to be extracted are not specifically limited.

First, according to an embodiment of the disclosure, a chamber for centrifugal separation is prepared (see reference sign (a) of FIG. 10). The chamber for centrifugal separation includes the chamber main body 105, the upper cover 120, the lower cover 130, the receiving space 110, the remaining liquid receiving space 180, and the filter device 140. In addition, the tube 150 is mounted on the chamber main body 105 and inserted into the lower injection port 131a of the lower cover 130.

When the chamber for the centrifugal separation is prepared, a density gradient material "A" is supplied to the tube 150. As described above, the density gradient material "A" supplied to the tube 150 is injected into the receiving space 110 (see reference sign (b) of FIG. 10) after passing the tube connector 165 and the lower injection port 131a.

Next, blood "B" is injected into the chamber main body 105 through the upper injection port 121a (see reference sign (c) of FIG. 10). The blood "B" may be injected by inserting a syringe or pipette into the upper injection port 121a. In this case, because the filter device 140 is provided inside the receiving space 110, the blood "B" and the density gradient material "A" may be prevented from being mixed with each other.

In this status, the centrifugal separation is performed after mounting the chamber for centrifugal separation in an apparatus for centrifugal separation. Accordingly, plasma "B3", PBMC "B2", the density gradient material "A", and the red blood cell "B1" are separated and sequentially positioned downward in the axial direction (see reference sign (d) of FIG. 11).

In this status, the density gradient material "A" is injected again into the receiving space 110 (see reference sign (e} of FIG. 11). As described above, the receiving space 110 includes the extracting part 115 having a smaller sectional area. Accordingly, the density gradient material "A" is supplied to the receiving space 110 to lift the PBMC "B2" to the position of the extracting part 115 such that the PBMC "B2", which serves as the target material to be extracted, is easily extracted. In this case, because the cap 170 is coupled to the lower injection port 131a, the density gradient material "A", which is supplied again, is prevented from moving toward the first opening 111a. Accordingly, even if the density gradient material "A" is injected again, the plasma "B3", the PBMC "B2", the density gradient material "A", and the red blood cell "B1" are prevented from being mixed with each other. In addition, even the filter device 140 may prevent the separated ingredients from being mixed with each other again. In detail, the filter device 140 may partially block a vortex, which is generated when the density gradient material "A" is injected, from being propagated to the boundary surface between the PBMC "B2", which is positioned above the density gradient material "A", and the density gradient material "A". Accordingly, the filter device 140 may prevent the PBMC "B2" from being mixed with another ingredient due to the vortex of the density gradient material "A".

However, an amount of PBMC "B2" contained in blood "B" is varied depending on persons. Accordingly, an amount of blood, which is to be injected into the receiving space 110 to extract a desired amount of PBMC "B2", is varied. Accordingly, even if the density gradient material "A" is injected to raise the PBMC "B2" to a desired position of the extracting part 115, the receiving capability of the receiving space 110 may be insufficient.

However, according to an embodiment of the disclosure, the chamber for the centrifugal separation includes the remaining liquid receiving space 180. Accordingly, when the density gradient material "A" is injected again, the plasma "B3" may flow over the receiving space 110 to be received in the remaining liquid receiving space 180 (see reference (e) of FIG. 1). Accordingly, even if a larger amount of blood "B" is required to extract the desired amount of PBMC "B2", the receiving space 110 may receive the blood "B".

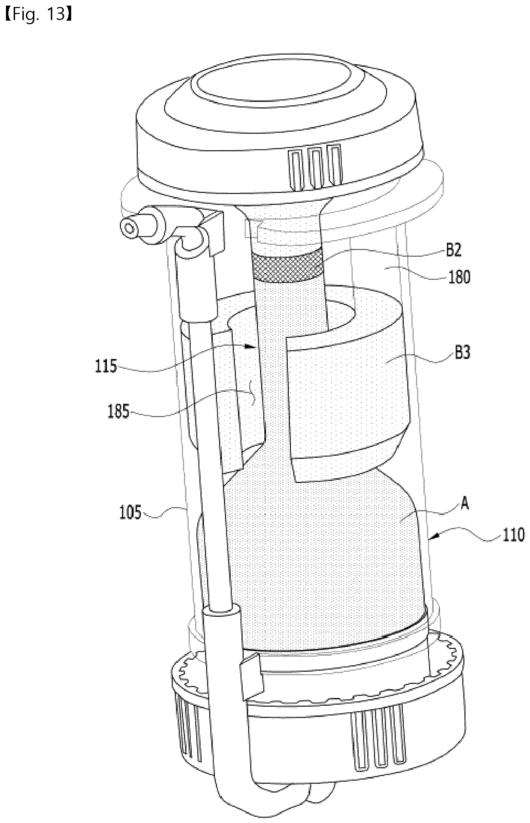

In addition, according to an embodiment of the disclosure, the chamber for the centrifugal separation may include the observing region 185 such that a user recognizes the height of the extracting part 115, at which the PBMC "B2" is positioned, with naked eyes of the worker. FIG. 13 is a perspective view illustrating a chamber for centrifugal separation, when extracting a target material, which is to be extracted, according to an embodiment of the disclosure. Referring to FIG. 13, even if the plasma "B3" is received in the remaining liquid receiving space 180, the extracting part 115 is exposed through a part having the observing region 185. Accordingly, a user may determine the position of the PBMC "B2" through the observing region 185.

Referring back to FIG. 11, the user may first extract the plasma "B3" positioned from the upper portion of the PBMC "B2" by inserting an extracting tool 200 into the upper injection port 121a while observing the extracting part 115 through the observing region 185 (see (f) of FIG. 11). In other words, even the position of the plasma "B3", which does not flow over the remaining liquid receiving space 180, may be determined. In addition, when the PBMC "B2" is extracted by first extracting the plasma "B3", the PBMC "B2" may be prevented from being mixed with another ingredient.

Accordingly, when the PBMC "B2" is positioned at a desired position of the extracting part 115, the PBMC "B2" may be extracted through the extracting tool 200 (see reference signs (g) and (h) of FIG. 12).

According to the chamber for the centrifugal separation of the disclosure, the remaining liquid receiving space is provided such that the target material to be extracted is positioned to the desired position, regardless of an amount of the target material for centrifugal separation. In addition, the target material to be extracted may be extracted while being determined with naked eyes of a user through the observing region. Accordingly, the target material may be more exactly extracted.

In addition, the density gradient material, which is injected before the centrifugal separation, is prevented from being mixed with the target material for the centrifugal separation due to the filter device, thereby preventing the target material to be extracted from being lost.

In addition, as the cap is coupled to the lower injection port, the material injected through the lower injection port is prevented from moving toward the upper opening, thereby preventing the separated ingredients from being mixed with each other, and more preventing the target material to be extracted from being lost.

Hereinabove, although the disclosure has been described with reference to exemplary embodiments and the accompanying drawings, the disclosure is not limited thereto, but may be variously modified and altered by those skilled in the art to which the disclosure pertains without departing from the spirit and scope of the disclosure claimed in the following claims. Therefore, embodiments of the disclosure are not intended to limit the technical spirit of the disclosure, but provided only for the illustrative purpose. The scope of the disclosure should be construed on the basis of the accompanying claims, and all the technical ideas within the scope equivalent to the claims should be included in the scope of the disclosure.

## Claims

1. A chamber for centrifugal separation, the chamber comprising:
a chamber body having an upper opening and a lower opening opposite to the upper opening;
an upper cover having an upper injection port and mounted on a chamber main body to cover the upper opening;
a lower cover having a lower injection port and mounted on the chamber body to cover the lower opening;
a receiving space provided in the chamber main body, and including a first receiving part communicating with the lower injection port and an extracting part extending upward from the first receiving part and having a section area smaller than a sectional area of the first receiving part; and
a remaining liquid receiving space provided in the chamber main body to receive a material flowing over the receiving space, as the material is injected into the receiving space from the lower injection port,
wherein the remaining liquid receiving space has an observing region to expose at least a portion of a region of the extracting part in a direction (extending direction) that the extracting part extends, when the extracting part is viewed from an outside of the chamber main body.

2. The chamber of claim 1, wherein the remaining liquid receiving space extends in the extending direction of the extracting part, and is formed to surround a partial region of the extracting part in a circumferential direction of the extracting part, and
wherein an observing region is formed in a remaining region, which is not surrounded by the remaining liquid receiving space, of the extracting part in the circumferential direction of the extracting part, in the chamber main body.

3. The chamber of claim 2, wherein the observing region includes a transparent material.

4. The chamber of claim 2, wherein the observing region includes a recess part recessed inward from the chamber main body.

5. The chamber of claim 1, wherein an inner surface of the remaining liquid receiving space in a radial direction is connected with an outer surface of the receiving space in the radial direction, and
wherein the outer surface of the remaining liquid receiving space in the radial direction is formed to protrude upward from the receiving space.

6. The chamber of claim 1, wherein the receiving space further includes:
a second receiving part extending upward from the extracting part and having a sectional area greater than a sectional area of the extracting part.

7. The chamber of claim 1, further comprising:
a filter device interposed between the upper injection port and the lower injection port, in the receiving space to prevent a material, which is injected through the upper injection port, from being mixed with the material injected through the lower injection port.

8. The chamber of claim 1, wherein the lower cover further includes:
a cap including a protruding part protruding inward of the first receiving part when the lower cover is coupled to the chamber main body, and having the lower injection port, and
wherein the cap is inserted in the lower injection port in an axial direction to prevent the material injected into the lower injection port from moving toward the upper opening,
when a direction parallel to the direction from the upper opening toward the lower opening is defined as the axial direction, and a direction perpendicular to the axial direction is defined as a radial direction.

9. The chamber of claim 1, wherein the lower cover includes:
a lower cover part to cover the lower opening; and
a lower side part extending toward the upper opening from a circumference of the lower cover part to cover an outer surface of the chamber main body, when the lower cover is mounted on the chamber main body, and
wherein the lower side part includes:
a roughness part having a repeated pattern formed along a circumference of an inner surface opposite to the outer surface of the chamber main body.

10. A method for centrifugal separation using a chamber for the centrifugal separation,
in which the chamber includes:
a chamber body having an upper opening and a lower opening opposite to the upper opening;
an upper cover having an upper injection port and mounted on a chamber main body to cover the upper opening;
a lower cover having a lower injection port and mounted on the chamber body to cover the lower opening;
a receiving space provided in the chamber main body, and including a first receiving part communicating with the lower injection port and an extracting part extending upward from the first receiving part and having a section area smaller than a sectional area of the first receiving part; and
a remaining liquid receiving space provided in the chamber main body to receive a material flowing over the receiving space, as the material is injected into the receiving space from the lower injection port,
wherein the remaining liquid receiving space has an observing region to expose at least a portion of a region of the extracting part in a direction (extending direction) that the extracting part extends, when the extracting part is viewed from an outside of the chamber main body.
